# EUROPEAN PATENT APPLICATION

(11) **EP 4 700 131 A1**
(43) Date of publication of application: **25.02.2026**
(21) Application number: 24195409.8
(22) Date of filing: 20.08.2024
(51) Int. Cl.: C12P 39/00, A61K 8/97

(54) **PROCESS OF PREPARING COSMETIC INGREDIENTS CONTAINING COSMETICALLY OR DERMATOLOGICALLY ACTIVE COMPONENTS FROM PLANT OR ALGAE**

(71) Applicant: Biorealis, S.r.o., 900 01 Modra (SK)
(72) Inventor: STREDANSKÝ, Miroslav, SK-900 01 Modra (SK); STREDANSKÁ, Silvia, SK-900 01 Modra (SK)
(74) Representative: Bachratá, Magdaléna

(57) **Abstract**

A combined process for a preparation of a cosmetic ingredient containing a cosmetically and/or dermatologically active component of plant or algae origin in an increased yield and at the same time postbiotics from beneficial microorganisms and a high content of oligosaccharide prebiotics with a negligible amount of monosaccharides is presented. It is based on a treatment of plant or algae biomass with an extrusion preferably combined with an ultrasonication and followed by enzymatic cleaving their structural polysaccharides by enzymes selected from cellulases, hemicellulases, xylanases and pectinases, in a combination with a fermentation with beneficial microorganisms, selected from bacteria and yeasts. The active component of plant or algae origin is preferably selected from the group consisting of phenolic compounds, vitamins, antioxidants, essential oils, hydrocolloids, proteins, peptides, terpenoids, amino acids, organic acids, and polyols. The fermented biomass obtained after the described treatment can be further processed by physical methods, such as heating, freezing, homogenization, filtration, centrifugation, concentration, drying or lyophilization.

## Description

### Field of the Invention

The present invention relates to the field of cosmetic and/or dermatological ingredients. In particular, the invention relates to cosmetic and/or dermatological ingredients obtained by physical pretreatment of plants and/or algae biomass using extrusion and ultrasonication which is followed by treatment with enzymes hydrolyzing their structural polysaccharides and fermentation with beneficial microorganisms.

### Background of the Invention

The cosmetic world is often changing their formulations to improve the products' quality and to attract new customers. People are more and more aware of their product selection and want to know about the ingredients of the products to secure them from chemicals perceived as toxic. So, cosmetic companies and researchers are in an urge to screen natural products for possible cosmetic applications. Although natural ingredients have been used for centuries for skin care purposes, nowadays they are becoming more present in formulations, due to consumers' concerns about synthetic ingredients/chemical substances. A dominant part of natural ingredients is represented by products obtained from plants and algae. The use of bioactive extracts or phytochemicals from a variety of botanicals in cosmetics accomplishes two functions: care of the body and to influence the biological functions of the skin, providing the nutrients for a healthy skin (Ribeiro AS et al., Cosmetics 2015, 2, 48-65), (Dureja H.et al., Indian J Pharm 2005, 37, 155-159). Generally, plant and algae extracts are a rich source of vitamins, antioxidants, essential oils and oils, hydrocolloids, proteins, terpenoids and other bioactive compounds providing different functional properties (Dubey NK et al., Curr Sci 2004, 86, 37-41).

Fermentation process improves the quality of the active phytoextracts and also facilitates their absorption by the human body. The reason behind this increased absorption comes from the fact that fermentation breaks down ingredients into smaller molecules which may be more readily absorbed by the skin, thus bioavailable (Sivamaruthi BS et al., Int J App Pharm 10, 2018, 4, 31-34). Moreover, significant dermatological effects are shown also by cells or lysates of beneficial microorganisms, e.g. probiotic bacteria, and their metabolites (Chiba, K, Japanese J Lactic Acid Bacteria 18, 2007, 3, 105-112; Puebla-Barragan S and Reid G, Molecules 2021, 26, 1249). The use of probiotic microorganisms and fermented cosmetic ingredients for skin care has already been described in patents. Thus, document EP 2704704 describes probiotic bacteria and/or soluble metabolites of a probiotic bacteria and/or a cell lysate of a probiotic bacteria for use in the treatment of a disorder associated with Tight Junction function. EP 2859918 describes a cosmetic composition containing, as an active ingredient, a fermented product prepared by fermenting a ginseng berry extract with *Pleurotus ferulae* mycelium for anti-inflammation and atopy alleviation. EP 1344528 discloses a skin preparation for external application comprising a fermented product obtained by treating a bean extract with *Bifidobacterium* for preventing and improving skin roughness. EP 1722804 describes a *Lactobacillus* extract or active fraction thereof for maintaining a healthy level of skin flora. A method for the treatment of acne by a fermentation product derived from a *Leuconostoc* bacterium is described in US 10159708. US 2003/021945 describes the use of yeast strain *Zygosaccharomyces rouxii* fermented products as an antimicrobial agent or as an antioxidant in food and cosmetics. WO 2013/025986 relates to topical compositions comprising as a cosmetic and/or pharmaceutical active component a yeast ferment of the algae *Gracilaria spp.* for stimulating proteasome activity of skin cells.

On the other hand, most of beneficial microorganisms, particularly probiotic bacteria and yeasts, exhibit a limited growth and metabolite production when cultivated on plant or algae derived substrates, such as lignocellulose. The main reason is a lack, or very limited production, of enzymes able to hydrolyze structural polysaccharides of plant and algae, such as cellulases, hemicellulases, pectinases (Tarraran L and Mazzoli R, FEMS Microbiol Lett, 365, 2018, fny126; Oh EJ and Jin Y-S, FEMS Yeast Res, 20, 2020, foz089; Goldbeck R et al., African J Biotechnol 11, 2012, 53,11595-11603). However, chemical, physicochemical and enzymatic pretreatment of the plant or algae biomass allows the hydrolysis of the structural polysaccharides. In particular, the application of the enzymes degrading the structural polysaccharides before or during the fermentation with bacteria and yeasts led to conversion of lignocellulosic biomass to valuable products, such as lactic acid (Tarraran L and Mazzoli R, FEMS Microbiol Lett, 365, 2018, fny126; Alrumman SA, Brazilian J. Microbiol 47, 2016, 110-119), ethanol (Oh EJ and Jin Y-S, FEMS Yeast Res, 20, 2020, foz089), fermented beverages (Toy JYH et al., Crit Rev Food Sci Nutr 2020, Nov 30,1-22). A combination of enzyme hydrolysis and fermentation for the preparation of cosmetic ingredients was described in several patent applications (KR20160047757A, CN113827523A, JP2016084303A, JP2009179643A, JP2019034899A, CN113332354A, CN109260076A, CN111317694A, CN113893310A, CN107898718A, JP2012077035A).

Recently, the enzyme hydrolysis of the lignocellulose biomass is used in the production of various novel oligosaccharide prebiotics, such as xylo-oligosaccharides, arabinoxylo-oligosaccharides, arabinogalacto-oligosaccharides, mannano-oligosaccharides, pectin-oligosaccharides, cello-oligosaccharides, rhamnogalacturono- oligosaccharides (Saini R et al., Bioengineered, 13, 2022, 2139-2172; Bhatia L et al., Preparative Biochem Biotechnol, 2019, 1-15; Valladares Diestra KK et al., Foods, 12, 2023, 1-20). The oligosaccharide prebiotics exhibit proven dermatological positive effects (Lolou L and Panayiotidis MI, Fermentation, 5, 41, 2019, 1-17). The oligosaccharide production from lignocellulose biomass requires its pretreatment prior to enzymatic hydrolysis, such as physical (hydrothermal, steam explosion, microwave) and chemical (alkaline or acid pretreatment, ozonolysis, and organic solvents). However, high temperatures (150-200 °C) and the presence of strong chemicals lead to the destruction of other valuable active substances (e.g. cosmetically or dermatologically active) in plants and algae during the pretreatment. Makaravicius T et al. (Catalysis Today 196, 2012, 16-25) used an extrusion process for pretreatment of wheat and rye biomass for the enzymatic production of arabinoxylo-oligosaccharides, but the temperature of the process was still high (110 °C). The extrusion is generally an efficient lignocellulose biomass pretreatment method (Konan D et al., Energies, 15, 9, 2022, 3002). Similarly, ultrasonication techniques allow the pretreatment of the plant biomass without the use of chemicals and high temperatures. The combination of ultrasound energy with a suitable reaction time, temperature and solvent contributes to the destruction of recalcitrant lignin structure, allowing the biomass to be used in biological process (Sharma S et al., Biomass Conversion and Biorefinery, 12, 2022, 1393-1408; Flores EMM et al., Ultrasonics Sonochemistry, 72, 2021, 105455). Another drawback during the hydrolysis of lignocellulose biomass to oligosaccharides is a simultaneous partial formation of monosaccharides. The presence of monosaccharides significantly reduces the quality of prebiotics. Their removal is technologically difficult e.g. (chromatography, nano-/ultrafiltration) and it significantly increases the production costs (Saini R et al., Bioengineered, 13, 2022, 2139-2172; Valladares Diestra KK et al., Foods, 12, 2023, 1-20; Miguez B et al., Frontiers in Chemical Engineering, 3, 2021, 1-17; Yan F et al., Grain&Oil Sci Technol, 5, 2022, 98-106).

The present patent overcomes two drawbacks in the current state of the art. The pretreatment of plant/algae biomass by extrusion at mild conditions preferably in combination with ultrasonication allows more effective enzymatic hydrolyze of the structural polysaccharides to the prebiotic oligosaccharides while preserving the plant/algae bioactive components. The following fermentation eliminates undesirable monosaccharides formed during enzymatic hydrolysis. Moreover, the combined process (extrusion, ultrasonication, enzymatic hydrolysis, fermentation) is associated with a complete release of the cosmetically or dermatologically active components from plant or algae and the formation of postbiotics by the beneficial microorganisms.

### Summary of the Invention

The present invention is based on the inventors discovery that the process of preparing the cosmetic or dermatological ingredient by the extrusion of plant and/or algae biomass at temperature lower than 80 °C preferably combined with ultrasonication and followed by enzyme treatment using hydrolases of structural polysaccharides (cellulases, hemicellulases, xylanases and pectinases) in combination with fermentation by beneficial microorganisms (bacteria and yeasts) allows obtaining the ingredients with a high content of cosmetically and/or dermatologically active components of plant or algae origin and at the same time containing oligosaccharide prebiotics from the plant and/or algae and postbiotics from the beneficial microorganisms. Undesirable monosaccharides formed during enzymatic hydrolysis are eliminated during fermentation by beneficial microorganisms. The resulting fermentation broth containing treated plant or algae mass can be successively processed by physical methods, such as heating, freezing, homogenization, filtration, centrifugation, concentration, drying and/or lyophilization. The obtained material in the form of solution, concentrate, powder, suspension, or homogenate optionally stabilized by preservatives and/or additives is suitable as a cosmetic or dermatological ingredient.

### Detailed Description of the Invention

The present invention is based on the fact that cosmetically and/or dermatologically active components of plants or algae are often bound to or enclosed in structural polysaccharides and they are released by extrusion preferably combined with ultrasonication which is followed by enzymatic cleaving these polysaccharides and fermentation with beneficial microorganisms. The treated plant or algae biomass is optionally processed by suitable physical methods and stabilized to obtain the final cosmetic or dermatological ingredient. When used herein, the term cosmetically and/or dermatologically active matters and cosmetically and/or dermatologically active components from plants or algae are the compounds having cosmetically and/or dermatologically positive effects known from prior art. Examples are phenolic compounds, vitamins, antioxidants, essential oils, hydrocolloids, proteins, peptides, terpenoids, amino acids, organic acids, and polyols.

The term "said plant" is the plant containing the cosmetically and/or dermatologically active matter known from prior art. Preferably, the said plant is selected from the group of *Abelmoschus moschatus, Achillea millefolium, Acmella oleracea, Acorus calamus, Actinidia deliciosa, Aesculus hippocastanum, Adansonia digitata, Agrimonia eupatoria, Alchemilla vulgaris, Allium ursinum, Aloe barbadensis, Aloysia citrioddora, Altea officinalis, Amorphophallus konjac, Ananas comosus, Angelica archangelica, Anthemis nobilis, Arabidopsis thaliana, Arctostaphylos uva-ursi, Arctium lappa, Argania spinosa, Arnica Montana, Artemisia drancunculus, Aspalathus linearis, Astragalus memranaceus, Avena sativa, Baptisia australis, Bellis perennis, Beta vulgaris, Betonica officinalis, Bertholletia excelsa, Borago officinalis, Betulla alba, Betulla varricosa, Betulla pendula, Brassica nigra, Calendula officinale, Camellia sinensis, Camellia oleifera, Cannabis sativa, Capsella bursa-pastoris, Cardamine matthioli, Carum carvi, Centaurea cyanus, Centaurium erythraea, Centaurium minus, Centella asiatica, Ceratonia siliqua, Cicoria comune, Cinnamonum cassia, Cinchona succirubra, Cinchona officinalis, Citrus aurantifilia, Citrus clementina, Citrus limon, Citrus paradisi, Citrus reticulata, Citrus sinensis, Citrullus vulgaris, Chelidonium majus, Chenopodium quinoa, Cnicus benetictus, Coffea arabica, Cola acuminata, Coriandrum sativum, Crataegus laevigata, Crocus sativus, Cucumis sativus, Cucurbita pepo, Cupressus, Curcuma longa, Cymbopogon nardus, Cymbopogon Citratus, Daucus carota sativa, Diospyros mespiliformis, Dipteryx odorata, Dispacus fullonum, Echinacea purpurea, Elettaria cardamomum, Equisetum arvense, Eucalyptus globulus, Eufrasia officiale, Fereula assa foetida, Filipendula ulmaria, Foeniculum vulgare, Fragaria x ananassa, Fragaria vesca, Galanthus nivalis, Gatega officinalis, Galega galeopsis, Galium verum, Genziana lutea, Geranium macrorrhizum, Gingko biloba, Glycyrhizza glabra, Hamamelis virginiana, Harpagophytum orocumbens, Hedera helix, Heliconia Curacao, Helichrysum arenarium, Helichrysum italicum, Hibiscus, Hippophae rhamnoides, Hordeum vulgare, Humulus lupulus, Hylocereus undatus, Hypericum perforatum, Hyssopus officinalis, Illicium verum, Inula helenium, Iris florentina, Jasminum officinale, Jasminum sambac, Juniperus communis, Lamium album, Laurus nobilis, Lavandula angustifolia, Lavandula stoechias, Lawsonia inermis, Lepidium meyenii, Lepidium sativum, Lens esculenta, Leucojum vernum, Levisticum officinale, Linum usitatissimum, Lycium barbarum, Lycopodium clavatum, Magnolia officinalis, Malva neglecta, Malva sylvestris, Matricaria chamomilla, Matricaria recutita, Medicago sativa, Melissa officinalis, Melilotus officinalis, Mentha arvensis, Mentha citrata, Mentha piperita, Mentha spicata, Menyanthes trifoliata, Mimosa tenuiflora, Moringa oleifera, Morus alba, Morus nigra, Musa paradisiana, Myristica fragrans, Myrtus communis, Nelumbo nucifera, Narcissus tazetta, Nymphaea alba, Ocimum basilicum, Olea europaea, Ononis spinosa, Opuntia Tuna, Origanum majorana, Origanum compactum, Origanum vulgare, Oryza sativa, Panax ginseng, Panicum miliaceum, Passiflora incarnata, Paullinia cupana, Petroselinum crispum, Persea americana, Phaseolus vulgaris, Phaseolus acutifolius, Phoenix dactylifera, Phyllostachys nigra, Physalis peruviana, Pimpinella anisum, Pinus, Piper methysticum, Piper nigrum, Pisum sativum, Plantago lanceolata, Plantago psyllium, Plukenetia volubilis, Polygonum multiflorum, Polygonum fagopyrum, Pongamia pinnata, Potentilla anserina, Primula veris, Prunus domestica, Prunus lannesiana, Prunus persica, Prunus spinosa, Pulmonaria officinalis, Punica granatum, Pyrus malus, Pyrus communis, Quercus robur, Quilaja saponaria, Raphanus sativus, Ribes nigrum, Rhaponticum carthamoides, Rheum rhabarbarum, Rhodiola rosea, Rosa canina, Rosa damascena, Rosa moschata, Rosa mallica, Rosa centifolia, Rosmarinus officinalis, Rubia tinctorum, Rubus idaeus, Rubus fruticosus, Ruta graveolens, Salicornia herbacea, Salix alba, Salvia Hispanica, Salvia officinalis, Salvia sclarea, Sambucus nigra, Sanicula europaea, Saponaria ocymoides, Saponaria officinalis, Satureja hortensis, Schizandra chinensis, Scutellaria alpina, Sesamum indicum, Secale cereale, Sigesbeckia orientals, Solanum lycopersicum, Stevia rebaudiana, Silybum marianum, Symphytum officinale, Syzygium aromaticum, Tabebuia impetiginosa, Tanacetum vulgare, Taraxacum officinale, Tephrosia purpurea, Tilia cordata, Thymus serpyllum, Thymus vulgaris, Tribulus terrestris, Trifolium pratense, Triticum vulgare, Tropaeolum majus, Tuber magnatum, Tuber melanosporum, Turnera diffusa, Urtica dioica, Usnea barbata, Vaccinium myrtillus, Vaccinium vitis-idaea, Vaccinium macrocarpon, Valeriana officinalis, Vanilla planifolia, Verbascum thapsiforme, Verbena officinalis, Vigna aconitifolia, Vigna angularis, Viola mammola, Viola cornuta, Viola tricolor, Viola arvensis, Viscum album, Veronica officinalis, Vitex agnus castus, Vitis vinifera, Wolfiporia extensa, Zea mays, Zingiber officinale.* However, this list is not limitative (only illustrative).

The term "said algae" refers to the algae containing the cosmetically and/or dermatologically active matter known from prior art. Preferably, the said alga is selected from the group of *Ahnfeltiopsis concinna, Ahnfeltiopsis durvillei, Ahnfeltiopsis leptophylla, Alaria crassifolia, Alaria crispa, Alaria esculenta, Alsidium corallinum, Ascophyllum nodosum, Bangia, Bifurcaria bifurcata, Bifurcaria rotunda, Botryococcus braunii, Bryopsis plumose, Bryothamnion triquetrum, Ceramium rubrum, Chaetomorpha antennia, Chlamydomonas hedleyi, Chlamydomonas nivalis, Chlorella fusca, Chlorella minutissima, Chlorella pyrenoidosa, Chlorella sorokiniana, Chlorella vulgaris, Chnoospora implexa, Chnoospora minima, Chondrocanthus acicularis; Chondrus canaliculatus; Chondrus crispus; Chorda filum; Cladophora; Cladosiphon okamuranus, Codium amplivesiculatum, Codium cuneatum, Codium fragile, Codium simulans, Codium tomentosum, Colpomenia peregrina, Colpomenia sinuosa, Corallina elongata, Corallina pilulifera, Corallina vancouverensis, Cyanidium Caldarium, Cystoseira barbata, Cystoseira foeniculacea, Cystoseira hakodatensis, Cystoseira osmundacea, Dictyopteris delicatula, Dictyota dichotoma, Dunaliella salina, Ecklonia cava, Ecklonia kurome, Ecklonia stolonifera, Eisenia arborea, Eisenia bicyclis, Entromorpha intestinalis, Enteromorpha linza, Eucheuma cottonii, Euchema spinosa, Fucus evanescens, Fucus vesiculosus, Ganonema farinosum, Gayralia oxysperma, Gelidiella acerosa, Gelidium cartilagineum, Gelidium crinaale, Gelidium robustum, Gigartina acicularis, Gigartina pistillata, Gracilaria verrucosa, Gracilaria gracilis, Gracilaria foliifera, Gracilaria salicornia, Gracilariopsis lemaneiformis;,Haematococcus pluvialis, Halidrys siliquosa, Halymenia durvillei, Halymenia floresia, Halymenia maculata, Halopteris scoparia, Himanthalia elongota, Hizikia fusiformis, Hydroclathrus clathratus, Hypnea musciformis, Ishige foliacea, Ishige okamurae, Isochrysis galban, Isochrysis litoralis, Isochrysis maritima, Jania rubens, Laminaria japonica, Laminaria ochroleuca, Laurencia caspica, Laurencia luzonensis, Laurencia obtusa, Laurencia pacifica, Laurencia rigid a, Macrocystis pyrifera, Meristotheca dakarensis, Nannochloropsis granulata, Nannochloropsis oculata, Osmundaria obtusilo, Padina concrescens, Padina gymnospora, Padina pavonica, Padina tetrastromatic, Palisada flagellifera, Palmaria palmata, Pelvetia canaliculata, Petalonia binghamiae, Planktochlorella nurekis, Porphyra haitanensis, Porphyra tenera, Porphyra umbilicalis, Porphyra yezoensis, Porphyridium aerugineum, Porphyridium cruentum, Porphyridium purpureum, Polysiphonia howei, Postelsiapal maeformis, Pterocladia capillacea, Pyropia columbina, Pyropia yezoensis, Rhizoclonium hieroglyphicum, Rhodella maculata, Rhodella reticulata, Rhodomela confervoides, Rosenvingea intrincata, Saccharina latissima, Sargassum fulvellum, Sargassum furcatum, Sargassum hemiphyllum, Sargassum fusiforme, Sargassum henslowianum, Sargassum horridum, Sargassum horneri, Sargassum muticum, Sargassum pallidum, Sargassum polycystum, Sargassum serratifolium, Sargassum siliquastrum, Sargassum thunbergi, Sargassum vulgare, Schizymenia dubyi, Skeletonema marinoi, Solieria chordalis, Stoechospermum marginatum, Tetraselmis chuii, Tetraselmis suecica, Tetraselmis tetrathele;, Turbinaria conoides, Turbinaria ornata, Ulva dactilifera, Ulva fasciata, Ulva lactuca, Ulva pertusa, Ulva prolifera, Ulva rigida, Undaria pinnatifida.* However, this list is not limitative (only illustrative).

Selected plant or algae is preferably used alone, optionally two or more plant or algae can be mixed before the enzyme treatment and fermentation. Any part of the plant or algae containing active matter can be used, e.g. root, haulm (stem, stalk), leaf, germ, sprout, shoot, bud, flower, branch, wood, bark, peel, fruit, stomata, xylem, phloem etc. Optionally the by-products of the agro-food production can be used. The said plant or algae is used in any suitable form, e.g. fresh, dried, frozen, and lyophilized etc. The said plant or algae can be also in the form of industrial and agro-food wastes or by-products.

According to the present invention, said plant or algae material is pretreated by extrusion and preferably followed by ultrasonication to facilitate the hydrolytic action of the enzymes. Optionally lipids in said plant or algae before the pretreatment are partially or completely eliminated, e.g. by pressing or extraction with a suitable solvent known from prior art.

According to the present invention, said extrusion is performed at the mild conditions, it means a low temperature (less than 80 °) and a short residence time (between 1 and 30 min). A suitable screw speed ranges from 20 to 400 rpm. Both single-screw extruders and twin-screw extruders can be used for said plant or algae material pretreatment. The moisture content of said plant or algae material (biomass) before the extrusion ranges from 8% to 55% according to the type of biomass. The preferred moisture is between 20% and 40%.

According to the present invention, said extrusion is preferably followed by ultrasonication at low frequencies. First, the extruded biomass is diluted by water and cooled to lower temperature, preferably in the range from 20 ° to 50 °. The moisture content of said plant or algae biomass before the ultrasonication is from 60% to 99% according to the type of biomass. The preferred moisture is between 75% and 95%. Applied operating frequencies can varied between 20 and 100 kHz depending on the biomass type. The preferred frequencies are in the range from 20 to 50 kHz. A delivered power should be at least 10 W L⁻¹.

According to the present invention, the enzymes cleaving the structural polysaccharides of plants and algae, specifically cellulose, hemicelluloses and pectins, by their at least partial hydrolysis are selected from the group of cellulases, hemicellulases, xylanases and pectinases. The examples are commercial RONOZYME^{®} MultiGrain, RONOZYME^{®}WX and RONOZYME^{®}VP from DSM, NATE-0118, NATE-0119, NATE-1202, EXWM-3943, DIS-1030, FJE-1431 and DIS-1023 from Creative Enzymes, Celluclast^{®}, Pectinex^{®} Ultra Mash, Panzea^{®}, Ultraflo^{®} Max and Ultraflo^{®} XL from Novozymes.

The said enzymatic reaction is performed in the presence of water under suitable conditions. Its purpose is not to obtain the complete polysaccharide hydrolytic products, such as easily fermentable sugars (monosaccharides, eventually disaccharides), but to produce partially hydrolyzed products (oligosaccharides) with a simultaneous release of the plant/algae cosmetically/dermatologically active components which are bound to or closed in said structural polysaccharides of the plant or algae biomass. When used herein, the term oligosaccharides present carbohydrate chains containing 3-10 monosaccharide units. The pretreated plant or algae biomass after the extrusion or ultrasonication is optionally diluted with water which is followed by pH adjustment. The obtained mixture after the enzyme addition is left at suitable temperature with or without stirring for the period necessary to break the structural polysaccharides preferably to oligosaccharides.

Said suitable conditions include the enzyme dosage, temperature, pH, time, water content, and optionally presence of enzyme activators. The suitable enzyme dosage, temperature and pH are chosen preferably within the recommended values range suggested by the enzyme producer and are specific for each enzyme preparation. The preferred temperatures are from 20 to 80 °C while the preferred pH values range from 3.5 to 9.0. The preferred enzyme reaction time is from 1 hour to 5 days. The preferred water content of said reaction mixture is from 75 to 99 % (w/w). However, these values are not limitative. Said enzyme activators are selected from the compounds containing Mg²⁺, Ca²⁺, Na⁺, Mn²⁺, Co²⁺, Ba²⁺, Zn²⁺ ferulate, tartrate, citrate, F-actin, nonionic detergents, etc. The enzymatic process of the present invention can be performed in one or more steps, depending on the type of plant or algae mass and the suitable conditions for the single applied enzymes. For example, when plant leaves are used as a starting material, the application of two enzymes (cellulase and hemicellulase) is preferred in a two-step process at two different conditions. When whole plant or algae is used as the starting material, the application of three enzymes (pectinase, cellulase and hemicellulase) is preferred in a three-step process at three different conditions. However, if the suitable working conditions of different enzyme preparations are similar, a one-step process is preferred. The plant or algae biomass after the enzyme hydrolysis is suitable as fermentation medium, directly or optionally after elimination of solids or optionally diluted with water or concentrated by techniques known from prior art.

According to the present invention, said fermentation is performed with beneficial microorganisms under suitable conditions in an appropriate container, preferably a fermenter / bioreactor. Said suitable beneficial microorganisms are selected from the group of microbial strains belonging to non-pathogenic bacteria (lactic acid bacteria, *Micrococcus, Corynebacterium, Acinetobacter, Cutibacterium, Brevibacterium, Bacillus subtilis, Bacillus coagulans, Bacillus longum, Nitrosomonas eutropha, Staphylococcus epidermidis, Vitreoscillafiliformis*) and yeasts known from prior art for their positive effect on the human skin. According to one embodiment of the present invention, said beneficial bacteria are lactic acid bacteria characterized by producing lactic acid as the major metabolic end-product of carbohydrate fermentation, such as the genera *Lactobacillus, Leuconostoc, Pediococcus, Lactococcus, Streptococcus, Aerococcus, Carnobacterium, Enterococcus, Oenococcus, Sporolactobacillus, Tetragenococcus, Vagococcus, Weissella, and Bifidobacterium.* Preferably, the selected lactic acid bacteria are the strains belonging to the genera *Lactobacillus* and *Bifidobacterium,* more preferably the strains of *Lactobacillus brevis, Lactobacillus casei, Lactobacillus paracasei, Lactobacillus plantarum, Lactobacillus acidophilus, Lactobacillus bulgaricus, Lactobacillus rhamnosus, Lactobacillus johnsonii, Lactobacillus fermentum, Bifidobacterium longum, Bifidobacterium lactis, Bifidobacterium bifidum, Bifidobacterium infantis, Bifidobacterium adolescentis, Bifidobacterium breve, Bifidobacterium animalis.*

According to another embodiment of the present invention, said beneficial non-pathogenic yeasts are selected from the strains belonging to the genus *Saccharomyces,* preferably *Saccharomyces cerevisiae* and *Saccharomyces boulardii,* the genus *Galactomyces,* the genus *Saccharomycopsis,* the genus *Zygosaccharomyces,* preferably *Zygosaccharomyces rouxii* and *Zygosaccharomyces bailii,* the genus *Kluyveromyces,* preferably *Kluyveromyces lactis* and *Kluyveromyces marxianus,* and the species *Schizosaccharomyces pombe, Lachancea thermotolerans, Metschnikowia ziziphicola, Torulaspora delbrueckii.*

Said microbial strain is preferably used alone in the form of a pure culture, optionally two or more strains can be applied in the form of a mixed culture, e.g. *Lactobacillus* with *Bifidobacterium, Lactobacillus* with *Lactococcus,* and *Lactobacillus* with yeast strain.

According to the present invention, said fermentation is performed in an appropriate container, preferably a fermenter/bioreactor, by an inoculation of the plant or algae biomass after the enzyme hydrolysis (fermentation medium) with said beneficial microorganisms at suitable conditions, such as pH, temperature and time. Before the inoculation, the fermentation medium is optionally fortified with small amounts of growth factors known from prior art, such as sugars, sources of nitrogen, vitamins, inorganic and organic salts and preferably pasteurized or sterilized to avoid undesirable contamination.

Said suitable pH and cultivation temperature depend on the type of the used microbial strain. The pH value is maintained between 2.5 and 8.0, preferably between 3.2 and 6.0. The temperature is maintained between 10 to 45 °C, preferably between 18 to 37 °C. The preferred cultivation time is from 6 hours to 21 days, more preferably from 20 hours to 7 days. The fermentation is anaerobic or aerobic and performed under stirring or without stirring. However, these values and conditions are not limitative (only illustrative).

According to another embodiment of the present invention, said enzymatic hydrolysis of structural polysaccharides of plant and/or algae and said fermentation with beneficial microorganisms is optionally performed simultaneously if the conditions of both processes, such as pH and temperature, are similar.

The fermentation broth obtained at the end of the fermentation containing treated plant or algae biomass is successively processed by physical methods known from prior art, such as heating, freezing, homogenization, filtration, centrifugation, concentration, purification, extraction, drying and/or lyophilization. The plant or algae materials obtained after that treatment with physical methods can be finalized in the form of solution, concentrate, powder, suspension, emulsion, or homogenate, optionally stabilized by preservatives commonly used in the cosmetic and dermatological fields. The obtained materials can be used directly as cosmetic or dermatological products or as cosmetic or dermatological ingredients. Optionally the obtained materials are mixed with other cosmetic or dermatological ingredients, such as vitamins, fragrances or essential oils, pigments or colorants, anti-caking agents, anti-foaming agents, fillers and bulking agents, thickeners, thickening agents, surfactants and emulsifiers, film-forming agents, propellants, pH adjusters and neutralizing agents, etc. Said obtained material can be applied in the production of soaps, shampoo, foams, lotions, ointments, sprays, creams, toners, gels, tinctures, masks, sera, sticks, etc.

### Examples

The invention is further illustrated by the following non-limiting examples:

### Example 1

### Combined treatment of horse chestnut (Aesculus hippocastanum) seeds

500 g of ground dried horse chestnut seeds were mixed with 350 ml of purified water and put into laboratory parallel twin-screw extruder Process 11 (Thermo Fisher Scientific). The extrusion was carried out at a temperature of 80 °C for 12 min at 300 rpm. The extruded biomass was transferred to the 5-L benchtop bioreactor Biostat^{®}B (Sartorius) and mixed with 3150 ml of hot water (80 °C) containing 0.8 g of magnesium sulfate and 1.0 g of polysorbate 80. The diluted biomass was cooled to 50 °C and pH was adjusted to 5.0. Then 8 ml of hemicellulase enzymes Ultraflo^{®} Max (Novozymes) and 8 ml of RONOZYME^{®} Multi Grain (DSM) were applied for 10 hours at gentle stirring. Subsequently 4 ml of pectinase enzyme Pectinex^{®} Ultra Mash (Novozymes) were added and the mixture was kept at 55 °C and pH 4.5 for 5 hours. After 25 min of pasteurization at 75 °C, this enzymatically treated biomass was inoculated with 60 ml of *Bifidobacterium longum* pure culture previously grown in BSM medium for 24 hours. The fermentation was performed anaerobically under gentle stirring at 37 °C and pH 4.5 for 4 days. The horse chestnut fermented medium was again pasteurized at 75 °C for 20 min and remaining solid matters were eliminated by filtration through kieselguhr layer. The obtained filtrate contained 7.78 g/l of the cosmetically and/or dermatologically active triterpene glycosides (expressed as anhydrous aescin and determined by TLC method) and 33.8 g/l of oligosaccharides (determined by HPLC method). The content of monosaccharides was 0.2 g/l (HPLC). Table 1 illustrates the recovery of aescin from horse chestnut seeds and the content of oligosaccharides and monosaccharides achieved after each operation (extrusion, enzyme treatment, and microbial fermentation) in comparison with a simple extraction at the same conditions (temperature, time, water content). All given concentrations are calculated for the final volume.

**Table 1**

| Operation | Aescin concentration (g/l) | Concentration of oligosaccharides (g/l) | Concentration of monosaccharides (g/l) |
|---|---|---|---|
| Simple extraction | 2.96 | 3.2 | 4.1 |
| Extrusion | 5.57 | 3.9 | 4.8 |
| Enzyme treatment | 7.83 | 35.9 | 17.9 |
| Fermentation | 7.78 | 33.8 | 0.2 |

### Example 2

### Combined treatment of wakame alga (Undaria pinnatifida)

450 g of chopped dried wakame alga were mixed with 250 ml of purified water and put into laboratory parallel twin-screw extruder Process 11 (Thermo Fisher Scientific). The extrusion was carried out at a temperature of 62 °C for 10 min at 250 rpm. The extruded biomass was then diluted with 1100 ml of water and cooled to temperature of 35 °C. The following ultrasonication was performed in the ultrasonicator UP400ST (Hielscher) at frequency 24 kHz and the power of 400W for 20 min. The obtained biomass was transferred in a 5-L benchtop bioreactor Biostat^{®}B (Sartorius) and mixed with 2200 ml of hot water (80 °C) containing 1.0 g of polysorbate 80, 20 g of glucose and 2 g of citric acid. The diluted biomass was pasteurized at 70 °C for 20 min. Then 8 ml of aseptically filtered hemicellulase Ultraflo^{®} Max (Novozymes) and 6 ml cellulase Celluclast^{®} were added after cooling to 37 °C and pH adjustment to 5.5. The mixture was immediately inoculated with 40 ml of *Lactobacillus plantarum* pure culture previously grown in MRS medium for 24 hours. The simultaneous enzyme treatment and fermentation was performed anaerobically under gentle stirring at 37 °C and at noncontrolled pH for about 12 hours. During this period, the pH value decreased to 3.5 due to the bacterial production of lactic acid. Then the simultaneous process continued for other 3 days at 37 °C and controlled pH 3.5. The wakame fermented medium was again pasteurized at 75 °C for 20 min and remaining solid matters were eliminated by centrifugation at 8,000 g. The obtained supernatant, after enzymatic treatment combined with fermentation, contained 18.8 g/l of the cosmetically and/or dermatologically active compound fucoidan (determined by toluidine blue assay measuring absorbance at 632 nm) and 23.1 g/l of oligosaccharides (determined by HPLC method). The content of monosaccharides was 0.2 g/l (HPLC). Table 2 illustrates the recovery of fucoidan from wakame achieved after each operation (extrusion, ultrasonication, simultaneous enzyme treatment and fermentation). All given concentrations are calculated for the final volume. The supernatant containing fucoidan was further processed by 5-fold concentration using rotary vacuum evaporator followed with spray drying to obtain fine powder suitable for cosmetic or dermatological use.

**Table 2**

| Operation | Fucoidan concentration (g/l) | Concentration of oligosaccharides (g/l) | Concentration of monosaccharides (g/l) |
|---|---|---|---|
| Simple extraction | 8.0 | 1.9 | 3.6 |
| Extrusion | 11.5 | 2.2 | 4.1 |
| Ultrasonication | 13,7 | 2.8 | 4.5 |
| Enzyme treatment and fermentation | 18.8 | 23.1 | 0.2 |

### Example 3

### Combined treatment of plantain (Plantago lanceolate) leaves

500 g of chopped dried plantain leaves were mixed with 300 ml of purified water and put into laboratory parallel twin-screw extruder Process 11 (Thermo Fisher Scientific). The extrusion was carried out at a temperature of 70 °C for 15 min at 350 rpm. The extruded biomass was then diluted with 1200 ml of water and cooled to temperature of 40 °C. The following ultrasonication was performed in the ultrasonicator UP400ST (Hielscher) at frequency 24 kHz and the power of 400W for 1 hour. The treated biomass was transferred to the 5-L benchtop bioreactor Biostat^{®}B (Sartorius) and mixed with 2000 ml of hot water (70 °C) containing 4 g of sodium chloride, 0.8 g of magnesium sulfate and 1.2 g of polysorbate 80. The diluted biomass was cooled to 50 °C and pH was adjusted to 6.0. Then 7 ml of hemicellulase RONOZYME^{®} MultiGrain (DSM), 5 ml of cellulase Celluclast^{®} (Novozymes) and 2.5 g of cellulase NATE-0118 (Creative Enzymes) were applied for 10 hours at gentle stirring. Subsequently pH was changed to 5.5 and temperature to 55 °C. The hydrolysis continued for 6 hours. After 20 min of pasteurization at 75 °C, this enzymatically treated biomass was inoculated with 60 ml of *Staphylococcus epidermidis* pure culture previously grown in tryptic soy broth for 24 hours. The fermentation was performed anaerobically under gentle stirring at 37 °C and pH 5.5 for 3 days. The plantain leaves fermented medium was again pasteurized at 75 °C for 20 min and remaining solid matters were eliminated by centrifugation at 6,000 g. The obtained supernatant contained 4.25 g/l of the cosmetically and/or dermatologically active compound aucubin (determined by HPLC method) and 30.9 g/l of oligosaccharides (determined by HPLC method). The content of monosaccharides was 0.2 g/l (HPLC). Table 3 illustrates the recovery of aucubin from plantain leaves and the content of oligosaccharides and monosaccharides achieved after each operation (extrusion, ultrasonication, enzyme treatment, and microbial fermentation) in comparison with a simple extraction at the same conditions (temperature, time, water content). All given concentrations are calculated for the final volume.

**Table 3**

| Operation | Aucubin concentration (g/l) | Concentration of oligosaccharides (g/l) | Concentration of monosaccharides (g/l) |
|---|---|---|---|
| Simple extraction | 2.13 | 0.9 | 3.3 |
| Extrusion | 3.51 | 1.2 | 4.0 |
| Ultrasonication | 3,97 | 1.4 | 4.3 |
| Enzyme treatment | 4.27 | 32.1 | 14.4 |
| Fermentation | 4.25 | 30.9 | 0.2 |

### Example 4

### Combined treatment of Robusta coffee (Coffea canephora) beans

600 g of green Robusta coffee beans were cooled with liquid nitrogen and finely grounded. The ground beans were mixed with 350 ml of purified water and put into laboratory parallel twin-screw extruder Process 11 (Thermo Fisher Scientific). The extrusion was carried out at a temperature of 80 °C for 20 min at 400 rpm. The extruded biomass was then diluted with 1050 ml of water and cooled to temperature of 40 °C. The following ultrasonication was performed in the ultrasonicator UP400ST (Hielscher) at frequency 24 kHz and the power of 400W for 30 min. The treated biomass was transferred to the 5-L benchtop bioreactor Biostat^{®}B (Sartorius) and mixed with 2.0 liter of hot water (75 °C) containing 4 g of sodium chloride, 0.8 g of magnesium sulfate, 2 g of citric acid and 1.0 g of polysorbate 80. The diluted biomass was cooled to 48 °C and pH was adjusted to 5.0. Then 8 ml of hemicellulase Ultraflo^{®} Max and 5 ml of cellulase Celluclast^{®} (Novozymes) were applied for 12 hours at gentle stirring. Subsequently pH was changed to 4.5 and temperature to 55 °C and the hydrolysis continued for 12 hours. After 25 min of pasteurization at 75 °C, this enzymatically treated biomass was inoculated with 20 ml of *Lactobacillus rhamnosus* pure culture previously grown in MRS medium for 24 hours. The fermentation was performed anaerobically under gentle stirring at 37 °C for 30 hours. During this period, the pH dropped to 3.30 due to the lactic acid production. Subsequently the medium was inoculated with 40 ml of *Saccharomyces boulardii* pure culture previously grown in barley wort for 36 hours. The fermentation continued in the bioreactor closed by a fermenting cock at 24 °C for 5 days. The coffee beans fermented medium was again pasteurized at 75 °C for 20 min and remaining solid matters were eliminated by centrifugation at 6,000 g. The obtained supernatant contained 18.9 g/l of the cosmetically and/or dermatologically active polyphenols (expressed as chlorogenic acid and determined by Folin-Ciocalteu assay) and 41.2 g/l of oligosaccharides (determined by HPLC method). The content of monosaccharides was 0.1 g/l (HPLC). Table 4 illustrates the recovery of polyphenols from green coffee beans and the content of oligosaccharides and monosaccharides achieved after each operation (extrusion, ultrasonication, enzyme treatment, and microbial fermentation) in comparison with a simple extraction at the same conditions (temperature, time, water content). All given concentrations are calculated for the final volume.

**Table 4**

| Operation | Polyphenol concentration (g/l) | Concentration of oligosaccharides (g/l) | Concentration of monosaccharides (g/l) |
|---|---|---|---|
| Simple extraction | 5.5 | 3.9 | 4.8 |
| Extrusion | 10.8 | 4.8 | 5.7 |
| Ultrasonication | 12,7 | 4.9 | 7.2 |
| Enzyme treatment | 17.3 | 45.1 | 28.8 |
| Fermentation | 18.9 | 41.2 | 0.1 |

### Example 5

### Combined treatment of bearberry (Arctostaphylos uva-ursi) leaves

450 g of chopped dried bearberry leaves were mixed with 280 ml of purified water and put into laboratory parallel twin-screw extruder Process 11 (Thermo Fisher Scientific). The extrusion was carried out at a temperature of 75 °C for 15 min at 350 rpm. The extruded biomass was then diluted with 1070 ml of water and cooled to temperature of 40 °C. The following ultrasonication was performed in the ultrasonicator UP400ST (Hielscher) at frequency 24 kHz and the power of 400W for 45 min. The treated biomass was transferred to the 5-L benchtop bioreactor Biostat^{®}B (Sartorius) and mixed with 2200 ml of hot water (70 °C) containing 4 g of sodium chloride, 0.8 g of magnesium sulfate, 2 g of citric acid and 1.0 g of polysorbate 80. The diluted biomass was cooled to 50 °C and pH was adjusted to 5.0. Then 6 ml of hemicellulase RONOZYME^{®} Multi Grain (DSM), 4 ml of cellulase Celluclast^{®} (Novozymes) and 2 g of cellulase NATE-0118 (Creative Enzymes) were applied for 10 hours at gentle stirring. Subsequently pH was changed to 5.5 and temperature to 55 °C. The hydrolysis continued for 6 hours. After 25 min of pasteurization at 75 °C, this enzymatically treated biomass was inoculated with 20 ml of *Lactobacillus paracasei* and 20 ml of *Lactococcus lactis* pure cultures previously grown in MRS and M17 media for 24 hours, respectively. The fermentation was performed anaerobically under gentle stirring at 32 °C and pH 4.5 for 4 days. The bearberry leaves fermented medium was again pasteurized at 75 °C for 20 min and remaining solid matters were eliminated by centrifugation at 6,000 g. The obtained supernatant contained 9.21 g/l of the cosmetically and/or dermatologically active compound arbutin (determined by HPLC method) and 27.9 g/l of oligosaccharides (determined by HPLC method). The content of monosaccharides was 0.2 g/l (HPLC). Table 5 illustrates the recovery of arbutin from bearberry leaves and the content of oligosaccharides and monosaccharides achieved after each operation (extrusion, ultrasonication, enzyme treatment, and microbial fermentation) in comparison with a simple extraction at the same conditions (temperature, time, water content). All given concentrations are calculated for the final volume.

**Table 5**

| Operation | Arbutin concentration (g/l) | Concentration of oligosaccharides (g/l) | Concentration of monosaccharides (g/l) |
|---|---|---|---|
| Simple extraction | 4.03 | 1.2 | 2.3 |
| Extrusion | 6.19 | 1.8 | 3.0 |
| Ultrasonication | 7,27 | 2.4 | 3.4 |
| Enzyme treatment | 9.07 | 29.1 | 13.9 |
| Fermentation | 9.21 | 27.9 | 0.2 |

### Example 6

### Combined treatment of blueberry (Vaccinium myrtillus) fruits and haulms

500 g of frozen blueberry fruits and 250 g of chopped dried blueberry haulms were mixed with 100 ml of purified water and put into laboratory parallel twin-screw extruder Process 11 (Thermo Fisher Scientific). The extrusion was carried out at a temperature of 70 °C for 15 min at 300 rpm. The extruded biomass was then diluted with 1150 ml of water and cooled to temperature of 35 °C. The following ultrasonication was performed in the ultrasonicator UP400ST (Hielscher) at frequency 24 kHz and the power of 400W for 40 min. The treated biomass was transferred to the 5-L benchtop bioreactor Biostat^{®}B (Sartorius) and mixed with 2000 ml of hot water (70 °C) containing 2 g of sodium chloride and 0.8 g of magnesium sulfate. The diluted biomass was cooled to 50 °C and pH was adjusted to 5.0. Then 3 ml of hemicellulase RONOZYME^{®} MultiGrain (DSM), 2 ml of cellulase Celluclast^{®} (Novozymes), 1.5 g of cellulase NATE-0118 (Creative Enzymes) and 2.25 ml of pectinase Pectinex^{®} Ultra Mash were applied for 10 hours at gentle stirring. Subsequently pH was changed to 4.5 and temperature to 55 °C. The hydrolysis continued for 10 hours. After 20 min of pasteurization at 75 °C, this enzymatically treated biomass was inoculated with 40 ml of *Bacillus coagulans* pure culture previously grown in tryptic soy broth for 24 hours. The fermentation was performed aerobically under gentle stirring at 37 °C and pH 5.5 for 3 days. The blueberry fermented medium was again pasteurized at 70 °C for 20 min and remaining solid matters were eliminated by centrifugation at 8,000 g. The obtained supernatant contained 2.18 g/l of the cosmetically and/or dermatologically active anthocyanins (expressed as cyanidin 3-rutinoside equivalents and determined spectrophotometrically measuring absorbance at 538 nm) and 35.9 g/l of oligosaccharides (determined by HPLC method). The content of monosaccharides was 0.1 g/l (HPLC). Table 6 illustrates the recovery of anthocyanins from blueberry fruits haulms and the content of oligosaccharides and monosaccharides achieved after each operation (extrusion, ultrasonication, enzyme treatment, and microbial fermentation) in comparison with a simple extraction at the same conditions (temperature, time, water content). All given concentrations are calculated for the final volume.

**Table 6**

| Operation | Anthocyanin concentration (g/l) | Concentration of oligosaccharides (g/l) | Concentration of monosaccharides (g/l) |
|---|---|---|---|
| Simple extraction | 0.83 | 1.1 | 14.3 |
| Extrusion | 1.40 | 1.8 | 17.4 |
| Ultrasonication | 1,77 | 2.7 | 18.1 |
| Enzyme treatment | 2.27 | 38.1 | 21.8 |
| Fermentation | 2.18 | 35.9 | 0.1 |

### Industrial applicability

The cosmetic ingredients obtained by the treatment of plant or algae biomass with extrusion preferably combined with ultrasonication and followed by enzymatic cleaving their structural polysaccharides in combination with fermentation by beneficial microorganisms according to the previous description, can be advantageously used for the production cosmetic preparations, such as soaps, shampoo, foams, lotions, ointments, sprays, creams, toners, gels, tinctures, masks, sera, sticks, etc. According to the beneficial microorganism and the type of plants or algae, in which there are active ingredients with different types of action, they can improve the properties of finished cosmetic products and manifest various benefits for the skin, such as moisturizing, brightening, regenerating, reducing oxidative stress, slowing down aging, reducing wrinkles and other anti-aging effects. The good compatibility with other commonly used cosmetic ingredients such as vitamins, fragrances and essential oils, pigments or dyes, anti-caking agents, anti-foaming agents, foaming agents, fillers and swelling agents, thickeners, surfactants and emulsifiers, film-forming agents, pH regulators and neutralizing agents makes simple their application in the manufacture of cosmetic products.

## Claims

1. A process for preparing the cosmetic ingredient containing cosmetically active components of a plant or an algae origin comprising the steps of:
a) an extrusion of the plant or algae material at temperature lower than 80 °C;
b) a treatment with enzymes selected from the group of cellulases, hemicellulases, xylanases and pectinases at 10-70 °C for 1-120 h;
c) a fermentation with beneficial microorganisms selected from the group of bacteria and yeasts at 10-45 °C for 2-240 h.

2. The process according to claim 1, **characterized in that** the plant or algal material after the extrusion step is subsequently treated with an ultrasonication at low frequencies from 20 to 100 kHz.

3. The process according to claim 1 or 2, wherein said beneficial yeasts are selected from the group belonging to the genus *Saccharomyces,* preferably *Saccharomyces cerevisiae* and *Saccharomyces boulardii,* the genus *Galactomyces,* the genus *Saccharomycopsis,* the genus *Zygosaccharomyces,* preferably *Zygosaccharomyces rouxii* and *Zygosaccharomyces bailii,* the genus *Kluyveromyces,* preferably *Kluyveromyces lactis* and *Kluyveromyces marxianus,* and the species *Schizosaccharomyces pombe, Lachancea thermotolerans, Metschnikowia ziziphicola, Torulaspora delbrueckii.*

4. The process according to claim 1 or 2, wherein said beneficial bacteria are selected from the group of lactic acid bacteria, *Micrococcus, Corynebacterium, Acinetobacter, Cutibacterium, Brevibacterium, Bacillus subtilis, Bacillus coagulans, Bacillus longum, Nitrosomonas eutropha, Staphylococcus epidermidis, Vitreoscilla filiformis.*

5. The process according to any one of claims 1 to 4, wherein said the plant or algae material is selected from the group of root, haulm, leaves, germs, sprouts, shoots, buds, flowers, branch, wood, bark, peel, fruit, stomata, xylem, and phloem.

6. The process according to any one of claims 1 to 5, wherein said microbial strain is used alone or in a combination with other said microbial strains.

7. The process according to any one of claims 1 to 6, wherein said enzymatic treatment is performed before said fermentation.

8. The process according to any one of claims 1 to 6, wherein said enzymatic treatment is performed simultaneously with said fermentation.

9. The process according to any one of claims 1 to 8, wherein the resulting fermentation broth is successively processed by physical methods selected from the group of heating, freezing, homogenization, filtration, centrifugation, concentration, purification, extraction, drying, and lyophilization.

10. A cosmetic composition containing the cosmetic ingredient obtained according to any one of claims 1 to 9.
